# EUROPEAN PATENT APPLICATION

(11) **EP 4 108 224 A1**
(43) Date of publication of application: **28.12.2022**
(21) Application number: 22180629.2
(22) Date of filing: 23.06.2022
(51) Int. Cl.: A61J 7/00, A61J 7/04

(54) **PILL DISPENSER AND HOME MEDICATION SYSTEM**

(30) Priority: 23.06.2021 US 202163214052 P; 22.06.2022 US 202217846689
(71) Applicant: Hussein, Fadi, Brookfield, WI 53045 (US)
(72) Inventor: Hussein, Fadi, Brookfield, WI 53045 (US)
(74) Representative: Barker Brettell LLP

(57) **Abstract**

A pill dispensing device including a body, member, and disc. The disc includes an aperture shaped so that the member can extend through the aperture, whereas the rotation of the member also rotates the disc. The disc includes first and second disc sections having a pill storage sections and a dispensing portion. The dispensing portion has an arm biased towards a stop, as well as a wheel. When the disc section is rotated, a wheel arm engages with the arm, causing the arm to rotate away from the stop and allowing the rotation of the disc section to continue in a first direction. The stop adjacent to the arm prevents the arm from rotating in a second direction and therefore prevents the disc section from rotating in a second direction. The rotation of the disc sections dispense medication from the device.

## Description

### FIELD OF THE INVENTION

The present invention relates to a system and method of dispensing pills using a pill dispensing device. The present invention more particularly relates to a home medication system including a pill dispensing device that is configured to alert and dispense medication according to a set medication type and dosage, as well as enable communicate between patients and healthcare providers regarding medication and/or dosage changes and compliance.

### BACKGROUND

Medication is used to treat or prevent illnesses or disease, and proper administration of medication requires appropriate dosage and timing. The dosage and timing may change according to a patient's age, weight, other medication, or health conditions. While medication may be beneficial and necessary for a patient, noncompliance to a medication regimen can cause adverse events. Compliance to a prescribed modification regimen is also important so that a suitable healthcare plan may be devised and adjusted as needed.

The main reasons for noncompliance are related to fatigue, loss of interest, memory issues and cognitive issues. Compliance with a prescribed medication regimen may be further complicated by frequent adjustments in medication types or dosages, causing confusion and either missed or wrong doses.

Devices, such as a typical pill box or container, are used to assist patients in complying with their prescribed medication. Those typical pill boxes include a series of seven containers - one for each day of the week. Pills may be placed into each container and a patient may ingest the pills within the day's container. While typical pill boxes may be beneficial to some patients, typical pill boxes may be too simplistic, do not offer sufficient customization, and do not adequately assist patients with more complicated medication regimes. Additionally, there are also home medication devices that also assist patients in complying with their medication. Those home medication devices are bulky, expensive, and inefficient. There is therefore a need for an affordable home medication dispenser device to assist patients complying with their prescribed medication.

### SUMMARY OF THE INVENTION

In one aspect, a method is disclosed of using a pill dispensing device or apparatus to alert patients at the appropriate time to take their medication and also dispense the proper medication and dosage. Once a physician prescribes a medication and its dosage, the information is transmitted to the pill dispensing device. The pill dispensing device is programed so that the pill dispensing device and/or paired mobile device alerts its owner at an appropriate time to take the medication.

Another aspect of the invention is a home medication system that will allow the medical team taking care of the patient to track medication compliance and allow for real-time adjustments in dosage. The device will send alerts to the physician's office and the pharmacy when refills and new prescriptions are needed. The device and paired mobile device also allow healthcare providers to download the medication dispensing log for review to assure compliance with taking their medications (e.g., heart medications, blood thinners, cancer medications, diabetes and hypertension medications).

These and other features and advantages of the invention will become apparent to those skilled in the art from the following detailed description and the accompanying drawings. It should be understood, however, that the detailed description and specific examples, while indicating preferred embodiments of the present invention, are given by way of illustration and not of limitation. Many changes and modifications may be made within the scope of the present invention without departing from the spirit thereof, and the invention includes all such modifications.

### BRIEF DESCRIPTION OF THE DRAWINGS

Preferred exemplary embodiments of the invention are illustrated in the accompanying drawings in which like reference numerals represent like parts throughout, and in which:
Fig. 1 is a perspective view of a pill dispensing device;
Fig. 2 is a side elevation view of the pill dispensing device of Fig. 1;
Fig. 3 is a front elevation view of the pill dispensing device of Figs. 1-2;
Fig. 4 is a top plan view of a tray of the pill dispensing device of Figs. 1-3;
Fig. 5 is a side elevation view of the tray of Fig. 4;
Fig. 6 is a perspective cut away view of a first embodiment of the pill dispensing device;
Fig. 7 is a cross sectional view of the first embodiment pill dispensing device of Fig. 6, taken across line 6-6 in Fig. 6;
Fig. 8 is a side elevation view of a wheel of a first embodiment of a disc in the first embodiment of the pill dispending device of Figs. 6 and 7;
Fig. 9 is a cross sectional view of the first embodiment of the disc of Fig. 7 taken across line 5-5 in Fig. 7;
Fig. 10 is a cut away view of the first embodiment of the pill dispensing device of Figs. 6 and 7, illustrating the disc of Figs. 8 and 9;
Fig. 11 is a perspective cut away view of a second embodiment of the pill dispensing device;
Fig. 12 is a perspective view of a second embodiment of a disc;
Fig. 13 is a plan view of a right-side of the second embodiment of the disk;
Fig. 14 is a plan view of a left-side of the second embodiment of the disk;
Fig. 15 is a schematic representation of a home medication system; and
Fig. 16 is a flowchart of a method of operating the device, represented in Figs. 1 and 11, using the home medication system, represented in Fig. 15.

### DETAILED DESCRIPTION

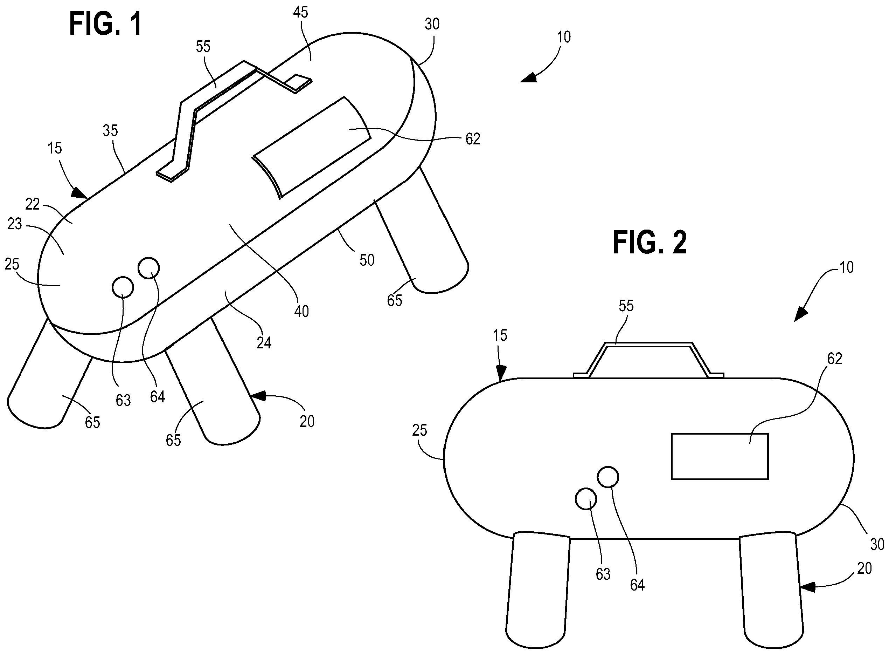

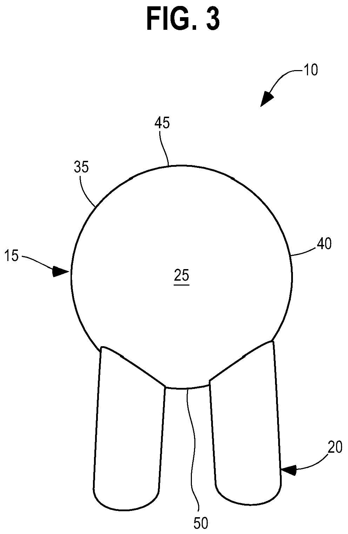

Referring now to Figs. 1-3, in accordance with an aspect of the invention, one embodiment of a pill dispensing device 10 includes a body 15 and a platform 20. The body 15 includes a housing 22 forming the shape of the body 15. As seen in the illustrated embodiment, the body 15 or housing 22 may cylindrical, but may be other shapes in alternative embodiments (e.g., spheroid or cuboid). The housing 22 may include a first housing portion 23 and a second housing portion 24, where both housing portions 23 and 24 extend from a first body end 25 towards a second body end 30. The first housing portion 23 may further be located at a top body side 45 and extends towards the bottom body side 50, while the second housing portion 24 is located at a bottom body side 50 and extends towards the top body side 45. The first and second housing portions 23 and 24 are further able to selectively engage each other through a snap fit, friction fit, interference fit, or hinged connection. Other engagement methods are envisioned and foreseeable. The body 15 further includes a first body side 35 and a second body side 40, located opposite from each other, and both further located between the first body end 25 and second body end 30.

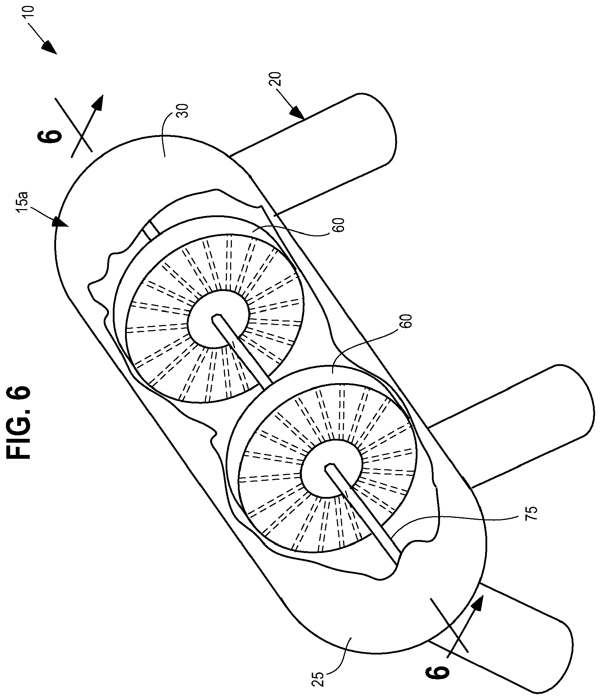

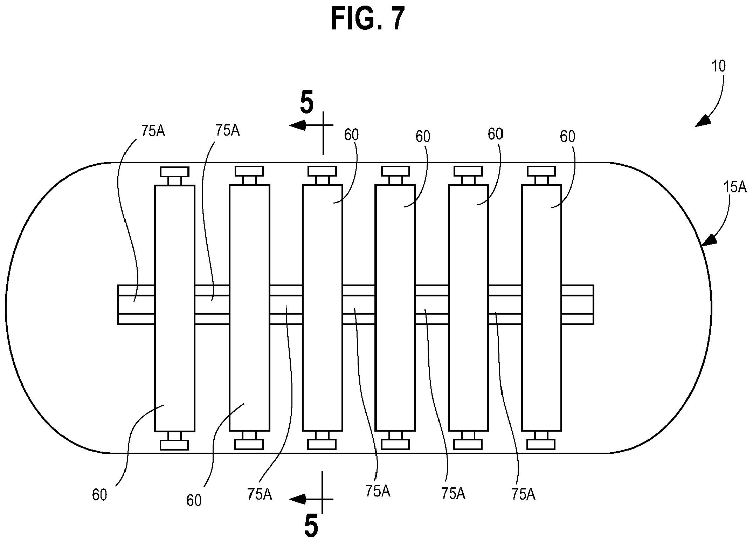

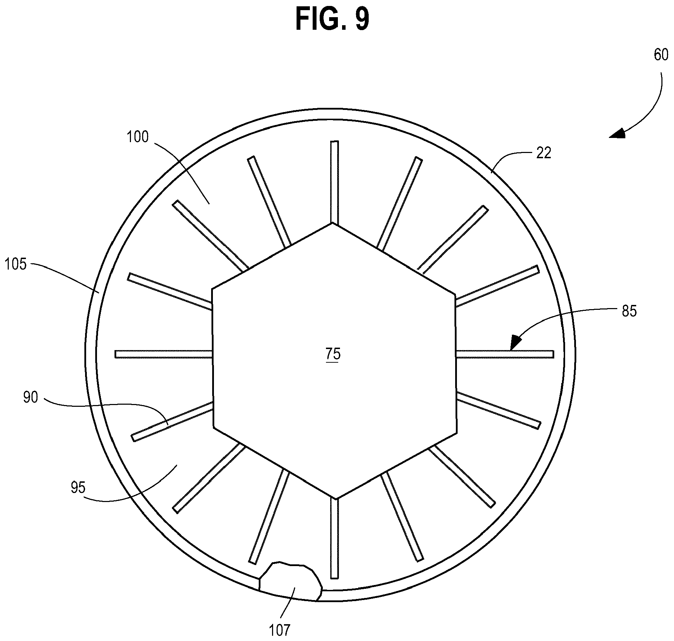

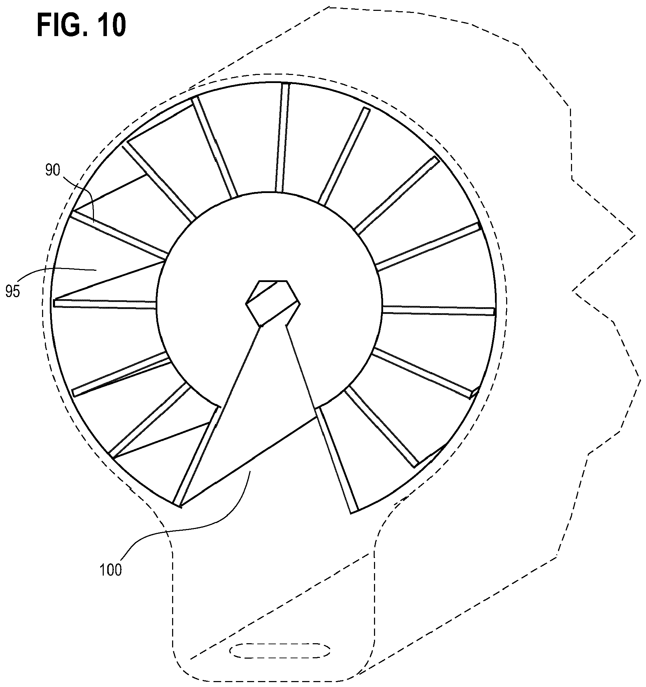

The pill dispensing device 10 may further include a handle 55 that projects away from the top body side 45. The handle 55 may be a loop in one embodiment. In other embodiments, the handle 55 may be any shape and size that allows a person may grip the handle 55 to carry the pill dispensing device 10 and/or assists in selectively disengage the first housing portion 23 from the second housing portion 24 to access at least one disc 60, 310 (shown in Figs. 6-14 and explained hereinafter) located within the housing 22. In an alternative embodiment (not shown), another method of accessing the at least one disc 60, 310 includes selectively disengaging a cap (not shown) located and positioned at the first body end 25 and/or second body end 30. The cap may selectively engage the housing 22 through a threaded fastener, snap fastener, friction fastener, etc. When the cap is removed from the housing 22, the at least one discs 60, 310 located within the housing 22, is selectively accessible and removable from the housing 22 to be refilled or exchanged as will be described hereinafter. In this embodiment, the housing 22 may not necessarily include the first housing portion 23 and the second housing portion 24. Instead, the selectively removable cap allows access to the interior of the housing 22.

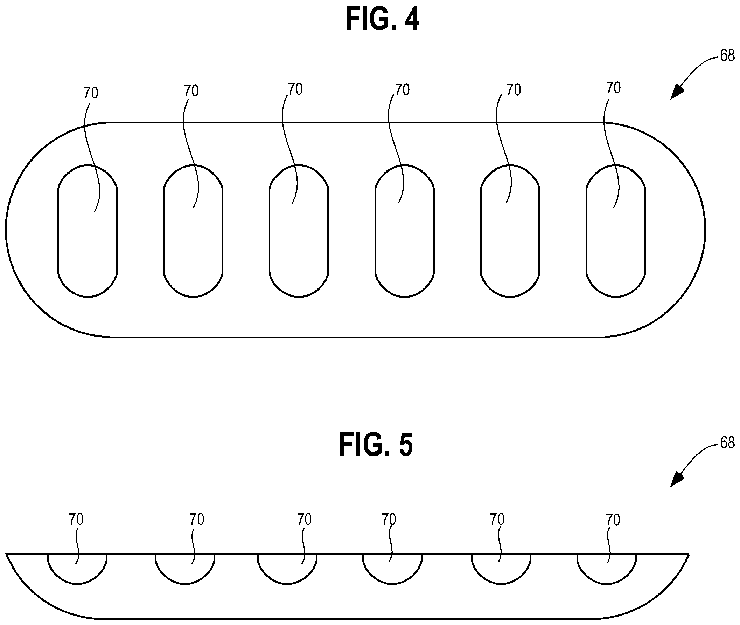

The platform 20 of the pill dispensing device 10 is located adjacent to the body 15, and projects away from the bottom body side 50 of the body 15. The platform 20 may be any structure that allows the bottom body side 50 of the body 15 to be spaced apart from a surface (not shown) on which the pill dispensing device 10 has been placed. As seen in Fig. 1, the platform 20 may be a series of legs 65, but may be other shapes, sizes, and components in other embodiments. Turning to Figs. 4 and 5, the pill dispensing device 10 preferably includes a tray 68. The tray 68 may be adjacent and abuts the body 15 in one embodiment, but may be located and positioned in the platform 20 in yet other embodiment. The tray 68 may be a member or platform that includes at least one well 70 shaped and sized so that at least one pill may fit within the at least one well 70. The tray 68 may be slidably attached to the body 15 (or platform 20) so that the at least one well 70 may be accessible such that a person can reach the dispensed pills within the well 70. The bottom body side 50 includes a series of apertures (not shown) that extends through the housing 22 allowing the at least one disc 60 to be in fluid communication with the at least one well 70 of the tray 68. In one embodiment, the series of apertures of the body 15 and/or at least one well 70 of the tray 68 correspond to the number and location of the discs 60, 310 within the pill dispensing device 10.

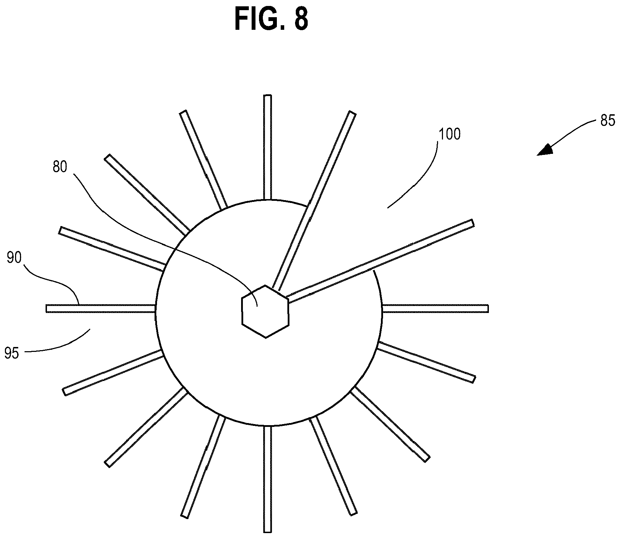

Describing a first embodiment of the components within a pill dispending device 10 and as seen in Figs. 6-10, the pill dispensing device 10 includes at least one disc 60 located and positioned within the housing 22 of the body 15A. The at least one disc 60 may be located on a rod or member 75 extending from the first body end 25 towards the second body end 30. The member 75 preferably has an octagon, hexagon, pentagon, rectangular, triangle, or other angled shape when viewed cross sectionally. The shape of the member 75 corresponds to the shape of the disc aperture 80 (shown in Fig. 8), allowing the member 75 to be locked or held in place within disc aperture 80, and therefore also rotates the disc 60 when the member 75 is rotated. Additionally, the member 75 is preferably formed from a series of member sections 75A where each member section 75A includes at least one motor (not shown), thereby allowing each member section 75A to rotate independently of its adjacent member section(s) 75A and also rotates the disc 60 engaged with that member section 75A. Each disc 60 can therefore also rotate independently of other discs 60.

As seen in Figs. 6, 8, 9, and 10, each of the at least one disc 60 further includes a wheel 85, with each blade 90 of the wheel 85 radiating out from the disc aperture 80. The two adjacent blades 90 form a pill storage section 95 in the empty space between the two adjacent blades 90, where at least one pill (not shown) may be placed within each pill storage section 95. The number of discs 60, as well as the number of blades 90 and pill storage section 95 within each disc 60, may differ according to the desired number of pills to be dispensed in the desired time period. For example, the desired time period may be a day, week, month, or months. The at least one disc 60 preferably also includes a blank section 100 where the blank section 100 may be a cutout or solid section where at least one pill cannot be placed within the blank section 100. In one embodiment, the blank section 100 may be shaped similarity to the pill storage section 95. The blank section 100 prevents any pills from falling out of the pill dispensing device 10 initially and also informs users that all the pills within that disc 60 have been dispensed, and the disc 60 must be replaced or refilled. Thus, the blank section 100 may initially be located adjacent to an aperture (not shown) of the body 15 so that no pills may be dispensed from the pill sections 95. Additionally, as the wheel 85 rotates, pills are dispensed from each pill section 95 until the disc 60 rotates back to the blank section 100, where no pills are dispensed thereby notifying the user that the disc 60 needs to be replaced or refilled. The at least one disc 60 may further include a disc casing 105. The disc casing 105 is preferably a shell or housing that substantially surrounds the wheel 85, leaving a cutout 107 shaped and sized similarly to the blank section 100 so that pills may be placed into and be dispensed from the disc 60.

Pills may be loaded into the disc 60 so that the appropriate number of pills (e.g., number of pills per scheduled dose) is placed with each pill storage section 95. Alternatively, a single pill may be placed within each pill storage section 95. The combination of the blank section 100 and the pill storage sections 95 allow the pills to be dispensed as needed. As will be explained in greater detail hereinafter, medication may be placed within each pill storage section 95, where the blades 90 prevent the pills within each pill storage section 95 from comingling with the pills in another pill storage section 95. Once the disc 60 has been filled with pills, the disc 60 may be loaded into the pill dispensing machine 10 and rotated so that the blank section 100 is adjacent to the cutout 107 of the disc casing 105 and corresponding aperture (not shown) of the body 15. When the pills are to be dispensed, the member section 75A rotates the wheel 85 within the disc casing 105 and housing 22, causing the pill(s) contained within each pill storage section 95 to move along an inner surface of the disc casing 105 until the pill(s) within a pill storage section 95 reaches the cutout 107 of the disc casing 105 and apertures of the body (not shown), whereas the pills may then fall from wheel 85, through the housing 22 of the body 15A, and into the tray 68. The tray 68 may then be removed from the pill dispensing container 10 or slid outwards to access the pills within the wells 70.

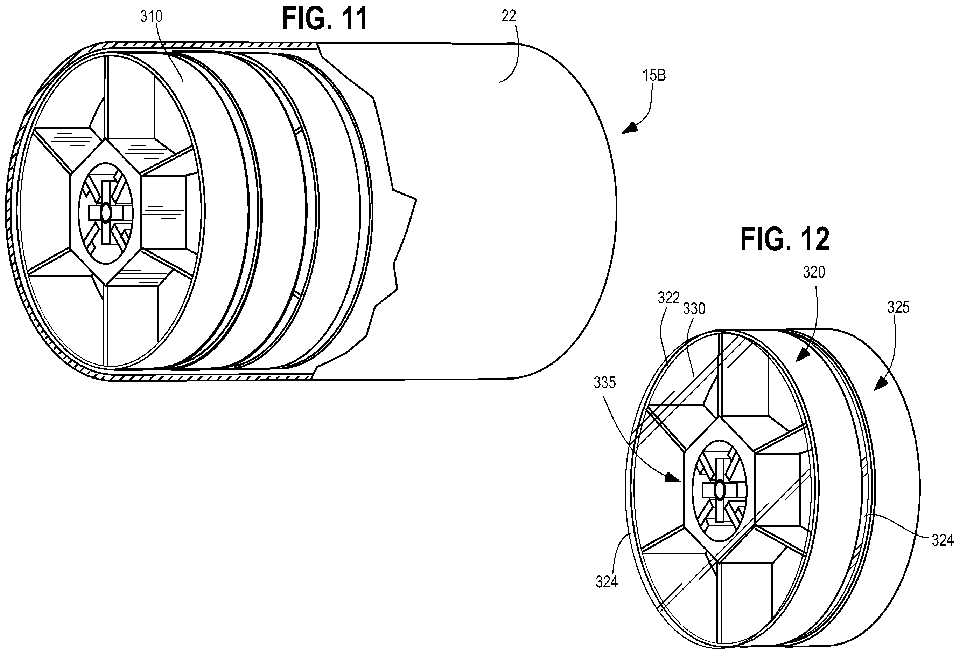

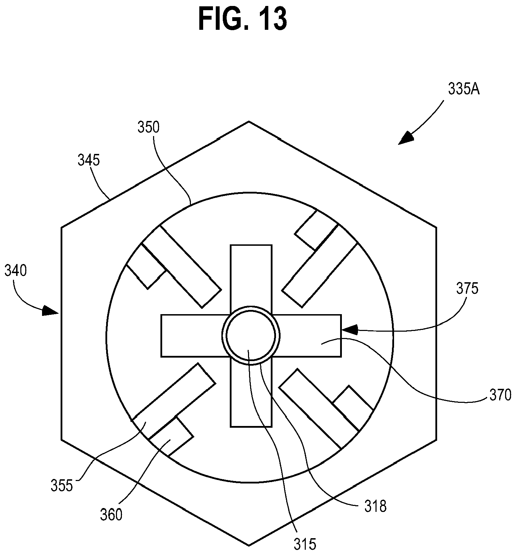

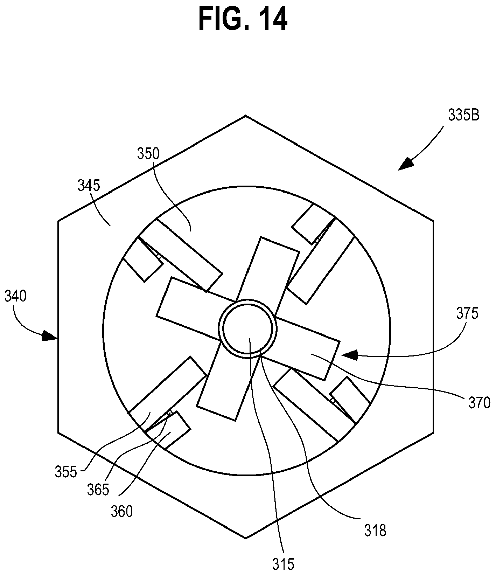

Describing a second embodiment of the body 15B of a pill dispensing device 10 and its components, and as illustrated in Fig. 11, the body 15B of a pill dispensing device 10 (shown in Fig. 1) includes a housing 22 that surrounds and encloses a series of discs 310. Similarly to the first embodiment of the body 15A, the second embodiment of the body 15B, also includes a rod or member 315 (shown in Figs. 13 and 14). The rod 315 extends horizontally or laterally through the pill dispensing device 10 and is preferably shaped so that the rod 315 (shown in Figs. 13 and 14) may be inserted into and/or through an aperture 318 of each disc 310. The second embodiment of the body 15B also includes at least one motor (not shown) that either turns the rod 315 and therefore also rotates the discs 310, or just rotates the discs 310. As described in more detail below, the rod 315 may also include rod section (not shown) similar to rod sections 75A described above. The rod sections may turn separately from one another, thereby allowing the discs 310 to turn or rotate independently from each other.

In one embodiment, and as seen in Fig. 12, each disc 310 includes a first disc section 320 and a second disc section 325. The first disc section 320 and second disc section 325 may be located and positioned adjacent to each other. Both the first disc section 320 and second disc section 325 include a tray 322. The tray 322 includes a series of pill storage sections 330 extending inwards from the circumference of each disc 310, as well as a cover 324 extending over the pill storage sections 330 to prevent pills from falling out of the disc 310. The first disc section 320 and second disc section 325 further include a dispensing portion 335. The dispensing portion 335 of the discs 310, illustrated in Figs. 13 and 14 as 335A and 335B, respectively, include a disc casing 340, which has both an outer casing wall 345 and an inner casing wall 350. The dispensing portions 335 of the discs 310 further include at least one arm 355 and at least one stop 360 which both extend from the inner casing wall 350. The arm 355 may be a rod or member, while the at least one stop 360 may be a protrusion or member located and positioned adjacent to the at least one arm 355. The first and second dispensing portion 355A, 355B further includes a spring 365 or other biasing method so that the arm 355 is able to selectively rotate away from the stop 360 when a wheel arm 370 engages the arm 355, and still return to its previous position - adjacent to the stop 360 - when the wheel arm 370 is no longer engaging the arm 355 (as will be discussed in more detail hereinafter).

As discussed above, the dispensing portion 355 also includes a wheel 375. The wheel 375 includes a wheel aperture 318 and at least one wheel arm 370 that projects away from the aperture 318. The aperture 318 is shaped and sized so that the rod 315 may be inserted into and/or through the aperture 318. The pill dispensing device 10 may therefore having a series of discs 310 within the pill dispensing device 10, with the rod 315 holding or suspending the discs 310 within.

In one embodiment, the at least one motor (not shown) engages the disc casing 340 and tray 322 to turn clockwise and/or anticlockwise. Alternatively, instead of the motor(s) rotating the pill storage sections 330 and disc casing 340, the rod 315 may be formed from a series of rod or member sections (not shown). Each member section of the rod may be connected to at least one motor (also not shown) which rotates the rod 315 clockwise and/or anticlockwise. Each member section of the rod 315 may further engage at least one disc section 320, 325 so that the at least one disc section 320, 325 also independently rotates when the motor rotates the member sections of the rod 315.

The location of the stop 360 in relation to the arm 355 on the first disc section 320 and the second disc section 325 prevents the first disc section 320 and second disc section 325 from turning either clockwise or anticlockwise. In more detail, because the stop 360 does not move, the arm 355 can only rotate away from the stop 360. Therefore, the first disc section 320 (shown in Fig. 13) can only rotate clockwise, while the second disc section 325 (shown in Fig. 14) can only rotate anticlockwise. Thus, when the pill dispensing device 10 is assembled with the series of at least one disc 310, with pills inserted into the tray 322 of both the first disc section 320 and the second disc section 325, the motor can rotate the disc 310 in one direction, allowing for a pill (or pills) to be dispended from a pill storage section 330 and into the well 70 of the tray 68. When the pill storage sections 330 are empty in either the first disc section 320 or second disc section 325, or at another set interval, the motor (or another motor) can rotate the disc 310 in the opposite direction thereby allowing for the pills in the other disc section 320, 325 to be dispensed. A greater number of pills or types of pills can therefore be stored and/or dispensed.

Referring back to Fig. 1, information about the medication regime may be displayed on a screen or display 62 (e.g., light emitting diode screen or a liquid crystal display), as illustrated in Fig. 1. The type of medication within the pill dispensing device 10 and their various dosages, the type of medication and dosage of that medication that is to be dispensed, current time, time remaining until the next scheduled dose, or other such information may be shown on the screen 62. The pill dispensing device 10 may also have an on/off switch or button 63 for powering on and off the pill dispensing device 10, as well as a dispensing switch or button 64 for dispensing the appropriate type of medication and dosage at a scheduled time or for a desired amount of time.

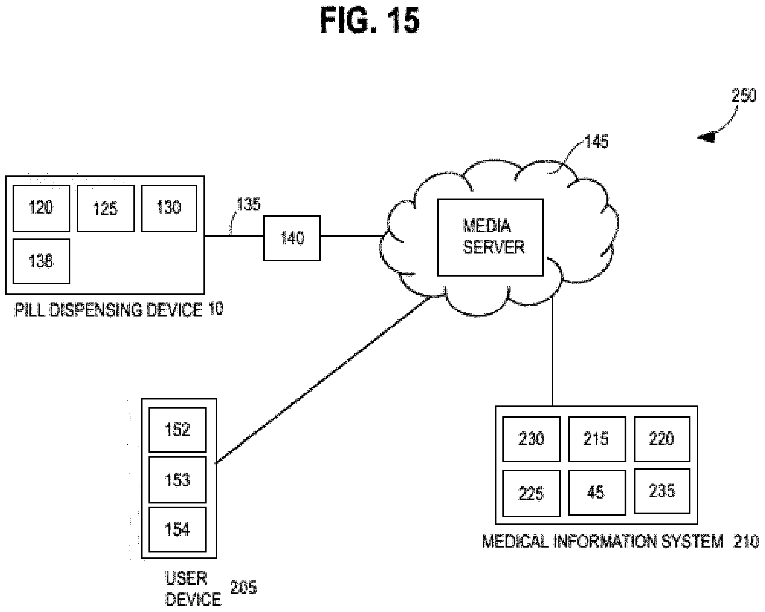

As illustrated in Fig. 15, the pill dispensing device 10 further includes a power supply (not shown) for powering the pill dispensing device 10. The pill dispensing device 10 may also include a processor 120, at least one memory storage 125, a wireless communication device 130, among other things. The pill dispensing device 10 can further include hardware, firmware, software, or any combination of above. The pill dispensing device 10 may communicate through a network such as a private Wireless Local Area Network (WLAN) 135, and is further connected to a Gateway and/or Router 140, processor 120 and/or non-transient memory storage 125. Pill dispensing device 10 can further include hardware, firmware, software, or any combination of above.

The controller 138 of the pill dispensing device may include control architecture having a processor 120 and memory 125. The controller 138 may have multiple processors 120, such as double processors and accompanying memory. Processors 120 can include any component or group of components that are configured to execute, implement, and/or preform any of the processes or functions described herein or any form of instructions to carry out such processes or cause such processes to be performed. Example of suitable processors include a microprocessor, microcontroller, and other circuitry that can execute software. Further examples of suitable processors include, but are not limited to, a central processing unit (CPU), an array processor, a vector processor, a digital signal processor (DSP), a field-programmable gate array (FPGA), a programmable logic array (PLA), an application specific integrated circuit (ASIC), programable logic circuit, and a controller. The processor 120 can include a hardware circuit (e.g., an integrated circuit) configured to carry out instructions contained in program code. The memory 125 stores one or more types of instructions and/or data. The memory 125 can include volatile and/or non-volatile memory. Examples of suitable memory 125 include random access memory (RAM), flash memory, read only memory (ROM), programable read-only memory (PROM), erasable programmable read-only memory (EPROM), electronically erasable programmable read-only memory (EEPROM), registers, disks, drives, or any other suitable storage medium, or any combination thereof. The memory 125 can be a component of a processor 120, can be operatively connected to a processor 120 for use thereby, or a combination of both. The memory 125 can include various instructions storage thereon. For example, the memory 125 can store one or more modules. Modules can be or include computer-readable instructions that, when executed by a processor 120, cause a processor 120 to perform the various functions disclosed herein. While functions may be described herein, it is noted that the functions are performed by the processor 120 using the instructions stored on or included in the various modules described herein. Some modules may be stored remotely and accessible by a processor 125 using, for instance, various devices and protocols.

In accordance with an aspect of the invention, a home medication system 250 for real-time alerts, dispersion, medication regime modifications, and monitoring can include a pill dispensing device 10, a user device 205, and medical informational system 210. The medical information system 210 has includes databases 230, processors 215, servers 220, hardware 225, software 45 such as Epic and Cerner, user interfaces 235, and other components used in a healthcare system. The home medication system 210 may further include at least one paired user device 205. User devices 205 may include such devices as a smart phone, tablet, laptop, or PC. The user device 205 may communicate with the pill dispensing device 10. Each user device 205 includes a display 153, internal computing and storage capabilities 152, and a program or application 154 that serves as a user interface with the remainder of the system 210.

The pill dispensing device 10 may communicate through a network such as a private Wireless Local Area Network (WLAN) 135, and is further connected to a Gateway and/or router 140. The pill dispensing device 10 may also be in communication with a server 145, which may be a cloud-sever. The server 145 can include or be coupled to a microprocessor, microcontroller or other programmable logic element (individually and collectively considered "a controller") configured to execute a program. Alternatively, interconnected aspects of the controller or circuitry and programs executed by it could be distributed in various permutations within the pill dispensing device 10, user device 150, medical information system 200, and server 145. The program may be used in processing, categorized, storing, recalling and transmitting data received from the pill dispensing device 10, paired user device 150, and/or medical informational system 200. It should be apparent that "circuity" in the regard can comprise hardware, firmware, software, or any combination thereof.

The pill dispensing device 10 may further communicate wirelessly with a paired mobile device. Information may therefore be transmitted over wi-fi from the pill dispensing device 10, paired mobile device 150, and/or medical information system 200. The paired mobile device 150 may have use applications as a user interface, where a patient, caretaker, or physician can review medical compliance, request the pill dispensing device 10 to dispense the proper medication for a set period of time if the patient has to be away from the pill dispensing device 10 (e.g., vacation). The paired mobile device 150 also receive alerts from the pill dispensing device 10 and notify its owner through flashing lights, vibration, or sound that it is time for the next dose of medication. The mobile device 150 may also send reminders a set time before the next scheduled dose of medication.

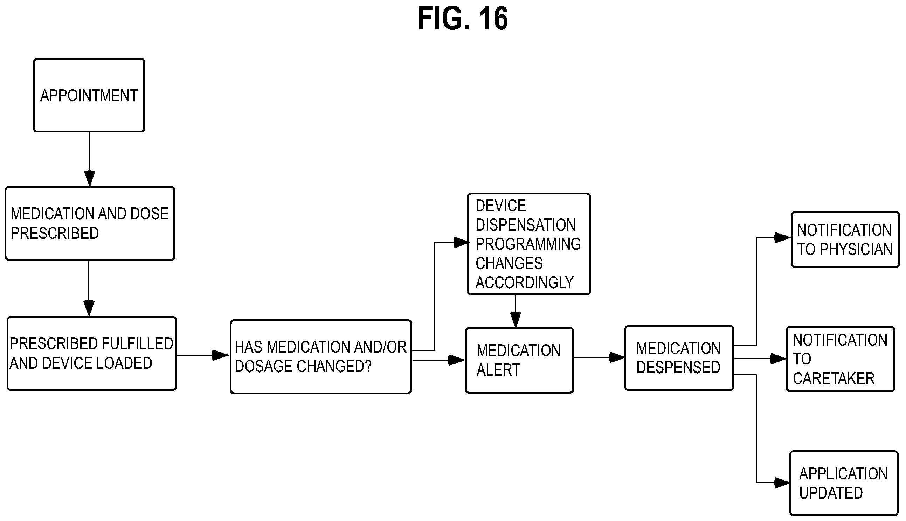

As shown in Fig. 16, in order to use the home medication system 250, the patient has an appointment with a physician where the physician prescribes the necessary medication for their treatment. Once the physician sets the medication regime, the device 10 may automatically pull the necessary information from the medical informational system 210 (e.g., taking the information from the patient's chart which may be stored in software such as Epic or Cerner) and further transmit that information to the paired user device 205 (or vice versa).

Once the prescription is fulfilled, the pill dispensing device 10 may be loaded with medication by the patient, caretaker, or pharmacist. In order to load the pill dispensing device 10 with medication, the first housing portion 23 and a second housing portion 24 may be selectively disengaged from each other (or the cap removed in alternative embodiments) to access the at least one disc 60, 310. The patient may receive their medication in a typical bottle from the pharmacist. The patient (or other person) may then fill the numbered pill storage sections 95 according to the instructions, which may be provided on the paired user device 205. In one embodiment, a single pill may be placed within each pill storage section 95 of the at least one disc 60, 310 so that only a single pill may be dispensed at a time when using the device 10. Alternatively, the empty discs 60, 310 may be removed and a pre-loaded at least one disc, purchased from a pharmacy, at home mailing service, or other locations possible to obtain medication, may be placed inside the pill dispensing device 10 replacing the previous discs 60, 310.

Once the pill dispensing device 10 is filled or loaded with the appropriate medication, the device 10 may send out alerts when it is time for the next dose. The pill dispensing device 10 may also send that information to the server 145, where it can then be transmitted to the paired mobile device 205. The paired mobile device 205 can also alert the patient of the next dose. Once the patient presses the dispense actuator 64, the wheel 85 or tray 322 within the pill dispensing device 10 rotates so that only the required mediation is dispensed (for example, if the patient requires 2 mg of a specific drug, the wheel 85 or tray 322 may rotate so that two 1 mg pills, each in a single pill storage section 95, 330 is dispensed) to ensure that the patient receives the correct dosage. Once the dispense button 64 is actuated, the device 10 sends a signal to the paired mobile device 305 to create a log showing what medication was taken and at what time. The patient's medical team may review the log to see if the patient was compliant in taking their medication. The patient's medical team may further receive an alert if the patient's lack of compliance endangers their health so that the medical team may follow up with the patient or caretaker. The device 10 may further send alerts to the physician and/or medical team and the pharmacy when a new prescription is required, and additional medication may be available for pick-up or sent to patient's home. If the medication regime changes, the device 10 may automatically update from the electronic medical records of the patient, allowing for real-time treatment adjustments.

Finally, the device 10 may dispense the appropriate amount of medication for a set period of time (e.g., the patient is traveling), so that the pills may be transferred to another container. For example, the user may transmit information to the device 10 (through the paired mobile device 205 or directly using the screen 62) that the user intends to travel for one week. Once the information is transmitted, the user may actuate the dispense button 64, and a week's worth of medication will be dispensed, whereas the patient may then transfer the mediation to another container.

Although the best mode contemplated by the inventors of carrying out the present invention is disclosed above, practice of the above invention is not limited thereto. It will be manifest that various additions, modifications and rearrangements of the features of the present invention may be made without deviating from the spirit and the scope of the underlying inventive concept.

It should be appreciated that such a development effort might be complex and time consuming, but would nevertheless be a routine undertaking of design, fabrication, and manufacture for those of ordinary skill having the benefit of this disclosure. Nothing in this application is considered critical or essential to the present invention unless explicitly indicated as being "critical" or "essential."

## Claims

1. A drug dispensing apparatus comprising:
a body having a member and an aperture;
at least one disc having at least two pill storage sections;
at least one motor controlling the rotation of the member;
wherein the member and the at least one disc selectively engage one another so that rotation of the member also rotates the at least one disc;
wherein the rotation of the at least one disc allows at least one pill to be released from a pill storage section and dispensed through the aperture.

2. The drug dispensing apparatus of claim 1 wherein the disc has a first disc section and a second disc section;
wherein the first disc section and second disc section are able to rotate independently from each other;
wherein each disc section includes at least one pill storage section.

3. The drug dispensing apparatus of claim 2 wherein each disc section includes a tray including at least one pill storage section, the tray being selectively rotatable within its disc section.

4. The drug dispensing apparatus of claim 2 or claim 3 wherein the first disc section and the second disc section both have a dispensing portion, each dispensing portion includes at least one stop and at least one arm;
wherein the at least one stop of the first disc section only allows the first disc section to rotate in one direction;
wherein the at least one stop of the second disc section only allows the second disc section to rotate in one direction.

5. The drug dispensing apparatus of claim 4 having one or more of the following features:
wherein the first disc section rotates in a first direction, wherein the second disc section rotates in a second direction;
wherein the at least one arm is biased so that its resting position is adjacent to the at least one stop;
wherein at least one spring biases the arm in an upright position, adjacent to the at least one stop.

6. The drug dispensing apparatus of claim any preceding claim wherein the member includes member sections, each member section being controlled by the at least one motor so that each at least one disc can rotate independently of another at least one disc.

7. The drug dispensing apparatus of claim 7 wherein at least one motor actuates the rotation of a first member section and therefore also actuates the rotation of the first disc section, wherein at least one motor actuates the rotation of a second member section and therefore also actuates the rotation of the second disc section, wherein the first disc section and second disc section of a disc rotate independently from one another.

8. A method of preparing a drug dispensing apparatus for use comprising:
filling at least one pill storage section of a first disc section and of a second disc section with medication;
placing a first cover over the first disc section, placing a second cover over the second disc section, the first and second covers preventing the medication from falling out of the at least one pill storage sections in the first disc section and the second disc section;
inserting the at least one disc into a drug dispensing apparatus.

9. A method of using a home medication system comprising:
obtaining at least one disc, the at least one disc have been filled with medication;
inserting the at least one disc within a drug dispensing apparatus;
pairing the drug dispensing apparatus with at least one mobile device and medical information system; and
using a controller in communication with the drug dispensing device to determine the amount and type of medication as prescribed; and
dispensing the medication according to a prescription.

10. The method of claim 9 wherein the home medication system further creates a log of drug dispensations for review to ensure medication compliance, optionally wherein the log of drug dispensations is uploaded to a patient's chart.

11. The method of claim 9 or claim 10, wherein the home medication system alerts a user that medication should be taken, optionally wherein the home medication system can remind users of an upcoming medication dose.

12. The method of claim 9 or claim 10 wherein the paired device can alert a user that the next dose of medication should be taken, optionally wherein the paired device can remind a patient of an upcoming medication dose.

13. The method of any of claims 9 to 12 wherein a user may request the drug dispensing apparatus to dispense the appropriate amount of medication for a future period of time.

14. The method of any of claims 9 to 13 wherein the drug dispensing apparatus and medical information system are in communication with each other such that changes to a patient's prescription may be automatically updated and reflected in drug dispensing apparatus.

15. A home medication system comprising:
a pill dispensing device for alerting and dispensing appropriate medication and dosages of that medication;
a controller in communication with the pill dispensing device, the controller including
a memory communicably coupled to the pill dispensing device and storing:
a database of medical records;
a medication type and dosage detecting module including instruction that, when executed, searches medical records for a prescribed medication regimen for a patient; and
an execution module including instructions that, when executed, execute a task.
